# EUROPÄISCHE PATENTSCHRIFT

(11) **EP 3 389 489 B1**
(45) Veröffentlichungstag und Bekanntmachung des Hinweises auf die Patenterteilung: **13.04.2022**
(21) Anmeldenummer: 16826019.8
(22) Anmeldetag: 16.12.2016
(51) Int. Cl.: A61B 5/07, A61B 5/03, A61B 5/00, A61B 5/01, A61B 5/11, A01K 11/00

(54) **VORRICHTUNG ZUM MESSEN DES MAGENDRUCKS UND DER MAGENMOTILITÄT EINES NUTZTIERES**
DEVICE FOR MEASURING THE GASTRIC PRESSURE AND THE GASTRIC MOTILITY OF A LIVESTOCK ANIMAL
DISPOSITIF DE MESURE DE LA MOTILITÉ DE L'ESTOMAC ET DE LA PRESSION DANS L'ESTOMAC D'UN ANIMAL D'ÉLEVAGE

(30) Priorität: 18.12.2015 DE 102015122293
(43) Veröffentlichungstag der Anmeldung: 24.10.2018
(73) Patentinhaber: Dropnostix GmbH, 14471 Potsdam (DE)
(72) Erfinder: BREITENSTEIN, Michael, 14478 Potsdam (DE)
(74) Vertreter: Schulz Junghans Patentanwälte PartGmbB
(86) Internationale Anmeldenummer: PCT/EP2016/081622
(87) Internationale Veröffentlichungsnummer: WO 2017/103239

(56) Entgegenhaltungen:
- WO-A1-2012/047150
- WO-A2-2011/079338
- DE-A1-102014 101 875
- US-A- 6 099 482
- US-A1- 2007 129 703
- US-A1- 2008 236 500
- LAWRENCE YU ET AL: "Chronically Implanted Pressure Sensors: Challenges and State of the Field", SENSORS, Bd. 14, Nr. 11, 31. Oktober 2014 (2014-10-31), Seiten 20620-20644, XP055333450, DOI: 10.3390/s141120620

## Beschreibung

Die Erfindung betrifft eine Vorrichtung zur dauerhaften, insbesondere ausschließlichen Erfassung des Magendrucks, der Magentemperatur, der Magenmotilität und der Bewegung eines Wiederkäuers gemäß Anspruch 1, sowie eine zweiteilige Vorrichtung zur Erfassung der Magenmotilität eines Wiederkäuers gemäß Anspruch 14. Des Weiteren wird ein Verfahren zum Messen des Magendrucks bei Nutztieren offenbart.

Die mit der modernen Landwirtschaft einhergehenden Produktionskrankheiten, wie beispielsweise die subakute Pansenazidose (SARA), sind ohne technische Hilfsmittel vorwiegend nur im Nachhinein oder indirekt feststellbar.

Besonders die SARA stellt ein ernstes gesundheitliches und ökonomisches Problem in der Landwirtschaft dar. So kann die SARA Auslöser einer reduzierten Futteraufnahme, reduzierten Milchleistung, Durchfallerkrankungen, Entzündungen der Pansenschleimhaut, Klauenkrankheiten (Klauenrehe, Sohlenblutungen und Sohlengeschwüren) sowie Lahmheit sein. Der finanzielle Verlust beläuft sich auf 400 EUR pro Kuh während der Laktation.

Die bisher bekannten technischen Lösungen zur Diagnose der SARA gehen den Weg über die Bestimmung des Pansen-pH-Wertes. Hierbei stehen zwei grundlegende Methoden zur Verfügung:
a) durch Probennahme des Pansensaftes, oder
b) durch kontinuierliche Messverfahren mittels eines im Pansen verbrachten Sensors.

- zu a) Die Probennahme erfolgt mittels Punktion des Pansen (Rumenozentese) unter Lokalanästhesie oder über eine oral eingeführte Schlund- bzw. Magensonde. Die hierbei ermittelten Werte sind jedoch methodisch bedingt mit Speichel vermengt, weshalb die Werte durchschnittlich um 0,5 pH-Einheiten (0,2 bis 1,9 pH-Einheiten) höher sind, als die mit Vergleichsmessungen durch Rumenozentese oder Fistel erhobenen Messungen. Der sich bei diesem Vorgehen ergebende Nachteil besteht darin, dass es sich um Ein-Punkt-Messungen handelt, welche ausschließlich von qualifiziertem Fachpersonal durchführbar sind. Bei der Rumenozentese kommt erschwerend hinzu, dass der operative Eingriff Nebenwirkungen haben kann und ein Infektionsrisiko für das Tier besteht. Ferner können bei letzterem auch Blutbeimengungen das Ergebnis beeinflussen.
- zu b) Hier wird wie in DE 60017916 T2, WO 2011/079338 A2, und GB 2455700 A ausgeführt ein Sensorsystem in Bolusform oral in den Pansen gegeben, um kontinuierlich den pH-Wert des Pansensaftes, telemetrisch zu übermitteln. Die Schwierigkeit besteht hierbei darin, dass die Sonde im Laufe der Zeit mikrobiologisch besiedelt wird und zunehmend ein eigenes Mikromilieu ausbildet, welches anstelle des zu messenden Pansensaftes gemessen wird. In der Labordiagnostik bedeutet ein Pansensaft-pH unter 6,0 eine akute Pansenazidose. Ist der pH für mehr als 3 Stunden am Tag kleiner als 5,6 kann von einer SARA ausgegangen werden. Der Umstand, dass auch gesundheitlich unauffällige Tiere pH-Werte aufweisen, welche definitionsgemäß eine Pansenazidose haben müssten, bringt eine gewissen Unschärfe in die bisherige Diagnostik.

Eine weitere Möglichkeit zur Detektion der SARA besteht in der Messung der Pansenaktivität und kann als indirekte Methode verstanden werden. Es ist bekannt, dass die SARA mit einer verminderten Pansenbewegung einhergeht. Gegenwärtig ist nicht abschließend geklärt, ob die reduzierte Pansenbewegung Ergebnis der pH-Wert-Veränderung ist oder sich letzteres infolge der Pansenbewegung ändert.

Generell besteht die Herausforderung darin, die eigentliche Pansenbewegung unter dem Hintergrund der Bewegung des betreffenden Tieres eindeutig und hinreichend genau zu isolieren. In der DE 29911803 U1 wird eine Vorrichtung zur Bestimmung der Magenmotilität beschrieben, welche durch ein piezoelektrisches Sensorelement die Bewegung des Magens erfasst. Diese hat jedoch den Nachteil, dass lediglich ein Verbiegen des Sensorelements auswertbare Signale erzeugt. Auch werden Druckänderungen im Organ nicht erfasst, sofern sie keine Verformung des Sensorelements bewirken.

Alternativ kann ein im Untersuchungsorgan befindlicher Beschleunigungssensor die Bewegung des Organs messen, allerdings erfasst ein solcher Beschleunigungssensor auch die Bewegungen des Tieres, so dass keine eindeutigen Rückschlüsse auf die Organbewegung gezogen werden können.

Ein weiterer Vorteil solcher intestinalen Bewegungsmessungen besteht darin, dass Langzeitmessungen möglich sind, die nicht von einem Biofilm bzw. mikrobiologischen Bewuchs beeinflusst sind, wie dies von pH-Messmethoden bekannt ist.

Weiterhin sei erwähnt, dass auch innerhalb der gleichen Tierpopulation große Schwankungen in den Zustandsparametern auftreten können und erst das Betrachten der Auffälligkeiten eines Individuums im zeitlichen Bezug für eine eindeutige Diagnose herangezogen werden kann.

Einige Vorrichtung aus dem Stand der Technik, sind insbesondere dazu ausgelegt, den Magendruck und/oder den pH-Wert im Magen des Tieres zu ermitteln, und die erfassen Sensordaten eine externe Empfängerstelle zu senden.

So offenbart beispielsweise US 2008/0236500 A1 eine Vorrichtung zum Erfassen des Magendrucks eines Wiederkäuers, wobei die Vorrichtung einen pH-Sensor und einen Drucksensor sowie ein Kommunikationsmodul aufweist, das dazu ausgelegt ist, die erfassten Sensordaten zu einem außerhalb des Nutztieres befindlichen Empfänger zu übertragen. Die Vorrichtung ist so ausgelegt, dass sie dauerhaft im Magen des Nutztieres verbleibt.

US 2007/0129703 A1 offenbart eine Kapsel zum Erfassen einer Kraft im Verdauungstrakt eines Säugetiers, wobei die Kapsel ein Gehäuse und eine an das Gehäuse angebrachte elastische Manschette aufweist, wobei die elastische Manschette so ausgebildet ist, dass sie einen gasdichten Innenraum formt. Ein Drucksensor ist so an der Vorrichtung angeordnet, dass er einen Druck im gasdichten Innenraum und damit einen Magendruck des Säugetiers erfassen kann. Des Weiteren umfasst die Kapsel ein Funkmittel zum Übertragen der Sensordaten.

DE 102014101875 A1 offenbart eine Vorrichtung zum Überwachen der Ernährung insbesondere einer Fermentation im Pansen eines Wiederkäuers. Die Vorrichtung umfasst einen pH-Sensor und eine Kommunikationseinheit zum Übermitteln der Sensordaten an eine Empfängereinheit. Weiterhin offenbart DE 102014101875 A1, dass die Vorrichtung ein spezifisches Gewicht von über 2,75 g/cm³ hat.

Ein weiterer wirtschaftlich relevanter Parameter ist das Erkennen der Brunstperiode. Die Zwischenkalbezeit, eine ökonomische Kennzahl für Milchwirtschaftsbetriebe, kann nur bei Kenntnis des optimalen Besamungszeitpunkts so kurz wie möglich gehalten werden. Wird der optimale Besamungszeitpunkt verpasst, so verlängert sich die Zwischenkalbezeit. Die heutigen Hochleistungsmilchkühe zeigen die Brunst schwächer ausgeprägt und zeitlich verkürzt an. Neben dem Duldungsreflex, welcher ein sicheres Brunstzeichen darstellt, ist während der Brunstperiode die Bewegungsaktivität der brünstigen Rinder etwa viermal höher als bei nicht-brünstigen.

Die Aufgabe der Erfindung ist daher, eine Vorrichtung bereitzustellen, mit der einerseits der Magendruck, die Magenmotilität und andererseits die Bewegungen eines Nutztieres über einen Zeitraum von mehreren Tagen zuverlässig ermittelt werden kann.

Das erfindungsgemäße Problem wird durch eine Vorrichtung der eingangs genannten Art gemäß Anspruch 1 gelöst. Vorteilhafte Ausgestaltungen der Erfindung sind in den Unteransprüchen angegeben und werden nachfolgend beschrieben.

Danach ist vorgesehen, dass eine solche Vorrichtung zur dauerhaften, insbesondere ausschließlichen Erfassung des Magendrucks, der Magentemperatur, der Magenmotilität und der Bewegung eines Nutztieres, insbesondere eines Wiederkäuers, einen Körper aufweist, der vorzugsweise bolusförmig ausgebildet ist, d.h. der Körper ist längserstreckt ausgebildet (in einer axialen Richtung) und weist zwei freie Enden auf, die in der axialen Richtung einander gegenüberliegen (ein solches Ende, an dem ein Drucksensor vorgesehen sein kann, wird hierin auch als oberes Ende bezeichnet), wobei jene Enden abgerundet sind (zur Verringerung einer Verletzungsgefahr), und wobei der Körper die folgenden Komponenten aufweist:
- eine telemetrische Einrichtung zum Übertragen von Messwerten,
- einen gasdichten Innenraum, wobei eine Wandung des gasdichten Innenraums bereichsweise ein elastisches Material oder einen elastischen Bereich aufweist, das bzw. der durch den Magendruck verformbar ist, und wobei der Drucksensor dazu ausgebildet ist, den Druck im gasdichten Innenraum zu erfassen, und wobei die Vorrichtung und insbesondere der Körper, so beschaffen ist, dass sie im Magen des Nutztiers dauerhaft verbleibt, wenn sie dem Nutztier appliziert wurde und insbesondere nicht durch Wiederkäuen oder Verdauung ausgeschieden wird. Dazu weist der Körper erfindungsgemäß eine mittlere Dichte von mehr als 2,3 g/cm³ auf.

Dauerhaft bedeutet in diesem Zusammenhang insbesondere, dass die Vorrichtung erst nach dem Tod oder durch operative Maßnahmen aus dem Magen des Nutztieres entfernt werden kann.

Die Erfindung ist weiterhin dadurch gekennzeichnet, dass die Vorrichtung einen Bewegungssensor umfasst, der insbesondere einen Beschleunigungssensor aufweist, wobei der Bewegungssensor dazu eingerichtet ist, die Bewegungsaktivität des Nutztiers zu erfassen.

Ein solcher Bewegungssensor, der die Gesamtbewegung des Tieres ermittelt, erlaubt durch die Protokollierung der ermittelten Bewegungsaktivitäten des Nutztieres Rückschlüsse auf die Brunst des Nutztieres, da, wie eingangs dargestellt, mit der Brunst eine veränderte Bewegungsaktivität des Nutztieres einhergeht.

Der Vorteil eines solchen integrierten Bewegungssensors ist, dass die Bewegung des Nutztieres unabhängig von etwaigen Fressbewegungen des Nutztieres (z.B. das Absenken des Kopfes beim Fressen) erfassbar ist, was bei einem Halsbandsensor nicht gewährleistet ist.

Wiederkäuer haben mehrere Mägen, die die Verdauung der Nahrung übernehmen. Als Magen bei einem Wiederkäuer wird in diesem Zusammenhang insbesondere, aber nicht notwendigerweise ausschließlich, der Pansen des Wiederkäuers bezeichnet.

Die Vorrichtung ist vorteilhafterweise bei Wiederkäuern einsetzbar insbesondere bei Rindern, insbesondere bei Milchkühen.

Die Vorrichtung kann beispielweise durch den Schlund des Nutztieres in den Magen des Nutztiers eingeführt werden. Dort kann sie den Magendruck sowie die Bewegungen des Magens, also die Magenmotilität erfassen, um dort über die beabsichtigte Laufzeit - relevant sind mindestens 60 Tage- Messwerte zu gewinnen. Eine längere Laufzeit kann durch eine entsprechend angepasste Energieversorgung erreicht werden.

Durch das Ersetzen chemischer Messmethoden durch rein physikalische Messungen (Pansen- bzw. Magenbewegung), die sich insbesondere dadurch auszeichnen, dass sie besonders stromsparend sind, können mit dieser Vorrichtung Laufzeiten von mehreren Monaten und sogar Jahren erreicht werden.

Wesentlicher Bestandteil der Vorrichtung ist der gasdichte Innenraum umfassend das elastischen Material bzw. den elastischen Bereich, das bzw. der sich infolge der von außen einwirkenden Kräfte verformen kann. Der Drucksensor ist dazu ausgebildet die dadurch resultierende Druckänderung im gasdichten Innenraum mittels geeigneter Sensoren, beispielsweise piezoelektrisch, resistiv, kapazitiv, optisch oder magnetisch zu detektieren, wobei dies falls notwendig mit einem Messwertumwandler geschehen kann.

Die Bewegung des Magens, in dem sich die Vorrichtung befindet oder das ihn umgebende Medium selbst, üben bei entsprechender Aktivität des Magens auf die Vorrichtung, im Konkreten das elastische Material, eine Kraft aus, die es verformt. Dadurch kommt es zu einer Druckänderung im gasdichten Innenraum, welche vom Drucksensor, erfasst wird.

Die Messdaten des Drucksensors können dann beispielsweise über eine elektrische Schaltung ausgewertet werden und an einen Mikrocontroller, in dem weitere Verarbeitungsschritte stattfinden, weitergeleitet werden. Die telemetrische Einrichtung ist dazu ausgebildet, die Messdaten oder auch eventuell verarbeitete Messdaten an einen Empfänger, der außerhabe des Nutztieres aufgestellt ist, zu senden. Dazu werden typische Frequenzbänder und Sendestärken verwendet, die für das Nutztier ungefährlich sind.

Der gasdichte Innenraum enthält ein Medium, beispielsweise ein Gas, ein Gasgemisch, ein Fluid, eine Lösung oder ein Gel.

In einer bevorzugten Variante weist das elastische Material bzw. der elastische Bereich ein blasenförmiges Elastomer (z.B. Latex oder Silikon) auf oder besteht aus dem blasenförmigen Elastomer. Die mechanische und milieuspezifische Stabilität des Körpers kann erreicht werden, indem der Körper beispielsweise POM (Polyoxymethylen), PVC (Polyvinylchlorid) oder PEEK (Polyetheretherketon) aufweist oder mit diesen oder anderen biokompatiblen Polymeren ummantelt ist.

Der Körper, oder ggf. kurz Bolus, kann mit einem Polymer (z.B. Epoxidharz) vergossen sein und ist vorzugsweise wasserdicht.

Der Körper ist beispielsweise pillenförmig.

Eine Vorrichtung mit diesen Merkmalen kann jegliche Krafteinwirkung im Magen eines Wiederkäuers, sei es durch Verformen (z.B. Verbiegen, Abknicken) oder statisch einwirkende Kräfte (z.B. langsam ansteigender oder abfallender Druck), erfassen. Der Vorteil der Bewegungsmessung bzw. Messung der Aktivität des Organs, in dem sich die Vorrichtung befindet, besteht darin, dass die Bewegung des Untersuchungsorgans weitgehend unabhängig von der Grundbewegung des Tieres ist.

In einer bevorzugten Ausführungsform der Erfindung weist der Körper eine mittlere Dichte von weniger als 3,0 g/cm³ auf, wobei die Dichte besonders bevorzugt bei 2,8 g/cm³ liegt.

Diese mittleren Dichten sind ausreichend hoch, um zu gewährleisten, dass die Vorrichtung dauerhaft im Magen des Nutztieres verbleibt.

In einer Ausführungsform der Erfindung weist der Körper ein Volumen zwischen 50 cm³ und 250 cm³, bevorzugt zwischen 70 cm³ und 160 cm³, auf, wobei das Volumen besonders bevorzugt 150 cm³ beträgt. Diese Größenbereiche gewährleisten in vorteilhafter Weise die Verträglichkeit der Vorrichtung im Magen des Nutztieres, insbesondere des Rindes, und gewähren gleichzeitig genügend Platz, um eine ausreichend große Energiequelle für die Elektronik, insbesondere für die telemetrische Einrichtung, im Körper (z.B. Bolus) zu installieren.

Die Energieversorgung und das Energiemanagement der erfindungsgemäßen Vorrichtung sind insbesondere derart ausgelegt, dass ein aktiver Betrieb der Vorrichtung, d.h. beispielsweise intermittierend oder periodisch stattfindende Messungen durch die Sensoren der Vorrichtung und telemetrische Übertragung der Messdaten an einen Empfänger, für einen Zeitraum von mindestens ein Jahr, möglich ist.

In einer bevorzugten Variante der Erfindung ist die Wandung des gasdichten Innenraums zumindest bereichsweise durch einen aus dem Körper herausragenden (oder von diesem abstehenden), elastischen Hohlkörper gebildet, wobei der Hohlkörper durch auf ihn einwirkende Kräfte im Magen des Nutztieres verformbar ist, wobei der Drucksensor dazu ausgebildet ist, eine Verformung des Hohlkörpers durch eine Druckänderung im gasdichten Innenraum zu erfassen. Eine solche Verformung kann beispielsweise durch die Bewegungen des Magens oder durch eine statische Druckänderung im Magen hervorgerufen werden. Dazu kann der Hohlkörper fest mit dem Drucksensor verbunden sein.

So gewährleistet die Kombination des Hohlkörpers als Sonde mit einem Drucksensor, dass die Aktivität bzw. die Bewegung des betreffenden Organs zuverlässig registriert werden kann.

Durch den hervorstehenden Hohlkörper wird in vorteilhafter Weise erreicht, dass neben dem Magendruck des Nutztiers auch die Magenmotilität des Nutztieres erfassbar wird.

In einer bevorzugten Ausführungsform der Erfindung ist der Hohlkörper durch Kräfte, die aus Bewegungen des Magens eines Nutztieres resultieren, knickbar, wobei der Hohlkörper dazu vorgesehen und ausgebildet ist, durch die diese Kräfte geknickt zu werden.

Ein Knick im Hohlkörper führt dementsprechend zu einem Drucksprung im gasdichten Innenraum, der vom Drucksensor erfassbar ist. Ein solcher Sprung im Druck ist in den Messsignalen deutlich registrierbar, und kann mit geeigneten Algorithmen besonders ausgewertet werden.

In einer weiteren Ausführungsform der Erfindung ist vorgesehen, dass der Hohlkörper schlauchförmig, insbesondere zylindrisch ausgebildet ist.

Diese Form des Hohlkörpers ist besonders geeignet, um einen knickbaren Hohlkörper zu gewährleisten. Je länger der schlauchförmiger Hohlkörper ausgebildet ist, desto leichter ist er knickbar, so dass die Vorrichtung durch verschiedene Längen des Hohlkörpers auf die jeweilige Nutztierart angepasst werden kann.

In einer weiteren Ausführungsform ist der elastische Bereich bzw. das elastische Material als Membran (z.B. kreisförmige Membran) ausgebildet, die über dem Innenraum liegt und diesen nach außen hin begrenzt bzw. abdichtet.

In einer weiteren Ausführungsform der Erfindung, weist der Körper ein Mittel auf, das dazu ausgebildet ist, den Hohlkörper oder die Membran lösbar mit dem Körper zu verbinden, so dass der Hohlkörper austauschbar ist, wenn die Vorrichtung beispielsweise bei einem weiteren Tier zum Einsatz kommen soll. Ein eventuell stark abgenutzter Hohlkörper/Membran kann so vergleichsweise einfach ersetzt werden, ohne dass der komplette Körper ausgetauscht werden muss.

Ein solches Mittel, kann beispielsweise durch eine Schraub- oder Klemmverbindung realisiert sein. Ein entsprechendes Verschlussmittel ist vorzugsweise ringförmig ausgestaltet.

In einer bevorzugten Ausführungsform der Erfindung weist die Vorrichtung einen Temperatursensor auf, der dazu eingerichtet ist, eine Magentemperatur im Nutztier zu erfassen, wenn die Vorrichtung in dem Magen des Nutztiers ist.

Ein Temperatursensor erlaubt es, Krankheiten oder gar den Kalbungszeitpunkt zu erkennen.

Dieser Temperatursensor kann Rückschlüsse auf das Trinkverhalten erlauben. Da das getrunkene Wasser zumeist kälter ist als der Pansen bzw. das Medium im Pansen, kommt es infolge der Wasseraufnahme zu einem Absinken der Temperatur im Pansen. Im zeitlichen Verlauf weisen die in einem Diagramm aufgetragenen Temperatur-Messwerte, während der Aufnahme von kälterem Wasser, somit nach unten gerichtete Peaks auf. Ist das Pansenvolumen bekannt, besteht anhand der Auswertung der Hysterese bzw. dem Temperaturverlauf zu einer Wasseraufnahme die Möglichkeit, die Menge an aufgenommener Flüssigkeit abzuschätzen bzw. anzunähern.

Insbesondere erhöht das korrelierte Auswerten von Bewegungsdaten und Temperaturdaten die Brunsterkennungsrate signifikant, so dass eine vergleichsweise deutlich zuverlässigere Aussage über die Brünstigkeit des Nutztieres getroffen werden kann, da die Brunst mit einer charakteristischen Temperaturkurve einhergeht.

Durch das isolierte Erfassen von Parametern, wie beispielsweise der Körpertemperatur allein, kann zwar beispielsweise eine Brunst oder eine Krankheit erkannt werden, ist aber nur eingeschränkt geeignet, präzise Aussagen über den genauen Zustand des Tieres zu treffen. Die Kombination von mehreren im Zusammenhang gemessenen Vitalparametern bei Nutztieren, wie zum Beispiel das Messen von Körpertemperatur, Körperbewegung bzw. Tieraktivität und die Pansenmotilität, insbesondere abgeleitet aus einer anisotropen Druckmessung im Verdauungsapparat des Nutztieres, erlauben eine differenziertere Beurteilung des Zustands des Tieres. Insbesondere durch die Korrelation oder Verknüpfung der Parameter Körpertemperatur, Bewegungsaktivität und Pansenmotilität, können diese im Zusammenhang stehenden Parameter dazu herangezogen werden, durch eindeutige Muster, sehr präzise Rückschlüsse auf beispielsweise tiergesundheitliche Zustände zu ziehen. So ist eine über drei Tage graduell abnehmende Körpertemperatur mit anschließendem Anstieg um 1 K, in Kombination mit einer über drei Tage graduell abnehmenden Pansenmotilität, sowie graduell über drei Tage steigender Tieraktivität, ein deutliches Indiz einer Brunst einer Kuh. Das Messen nur eines der vorgenannten Parameter allein kann nicht die Signifikanz der korrelierenden drei Parameter erreichen.

In einer weiteren Ausführungsform der Erfindung ist die telemetrische Einrichtung dazu eingerichtet, die Messdaten des Drucksensors und insbesondere die Messdaten des Temperatursensors und/oder des Bewegungssensors zu verarbeiten und die verarbeiteten Daten an einen Empfänger zu senden.

Der oder die Sensoren stehen in einer bevorzugten Ausführung in direktem Kontakt mit der telemetrischen Einrichtung des Körpers, welche bevorzugt mit Hilfe eines Mikrocontrollers die Ergebnisse entweder automatisch oder auf Anforderung bzw. durch Abfrage an eine Basisstation übermittelt. Die erhobenen Daten können in einer bevorzugten Ausführung mit Hilfe eines Computers ausgewertet bzw. dargestellt werden. Ebenso ist es möglich die Daten bereits auf dem Mikrocontroller in der Vorrichtung zu verarbeiten. Wobei der Mikrocontroller diese Daten insbesondere umrechnet, speichert, mit Zeitstempel versieht, und/oder an die telemetrische Einrichtung übermittelt.

Die Vorrichtung enthält eine eindeutige Kennung, welche entweder fest vorgegeben ist oder vom Anwender frei vergeben werden kann, wobei auch eine Kombination beider Varianten Anwendung finden kann. Erfindungsgemäß kann die Vorrichtung selbsttätig oder auf Abruf den Status der Vorrichtung und oder einen Alarm an der Basisstation anzeigen.

Bevorzugt werden die von dem Drucksensor, ermittelten Daten, und - falls vorhanden - auch die Daten des Bewegungssensors und des Temperatursensors, in einem in der Vorrichtung integrierten Speicher abgelegt und entweder in festgelegten Intervallen oder auf Abruf mit Hilfe der telemetrischen Einrichtung an die Basisstation übermittelt. Auf der Basisstation werden die Daten insbesondere von der beim Nutzer im Einsatz befindlichen Datenerfassungsvorrichtung gesammelt und visualisiert. Der Nutzer kann die auf der Basisstation zusammengeführten Daten abrufen und den zeitlichen Verlauf der Messdaten eines Tieres darstellen oder erfindungsgemäß den Vergleich zu einer Gruppe von Tieren vornehmen, um Rückschlüsse auf ihren Gesundheitszustand zu ziehen.

Die vom Sensor ermittelten Daten werden insbesondere entweder in festgelegten oder frei wählbaren Intervallen in einem mit der Vorrichtung verbundenen Speicher abgelegt und bei Bedarf entweder telemetrisch abgerufen oder telemetrisch an eine Basisstation übermittelt.

Die Daten aus der Basisstation, welche entweder stationär aufgebaut oder in Form eines Handgeräts ausgestaltet sein kann, können von einem Computer oder einem anderen für diese Aufgabe zweckdienlichen Vorrichtung verarbeitet und in einer bevorzugten Ausgestaltung derselben, visualisiert werden.

Gemäß einer weiteren Ausführungsform der Erfindung ist vorgesehen, dass der Körper einen ersten Bereich und einen zweiten Bereich aufweist, wobei der erste Bereich des Körpers radio- und funkwellenpermeabel ist, und wobei der zweite Bereich dazu ausgebildet ist, eine hinreichend große mittlere Dichte des Körpers zu erreichen.

Nur durch die Funkwellenpermeabilität des ersten Bereichs kann gewährleistet werden, dass die telemetrische Einrichtung die Messdaten aus dem Magen des Nutztieres herausfunken kann. Um nun eine ausreichend hohe mittlere Dichte des Körpers zu erreichen, ist es vorteilhaft den zweiten Bereich aus einem formstabilen, inerten, biokompatiblen und ausreichend dichten Materials zu fertigen, da die meisten funkwellenpermeablen Materialien eine zu geringe Dichte aufweisen, um eine mittlere Dichte der Vorrichtung zu erreichen, die hinreichen groß ist, ein Ausscheiden zu verhindern.

In einer Ausführungsform der Erfindung weist der erste Bereich einen insbesondere zylinderförmigen Mantel aus einem biokompatiblen Polymer auf, wobei die telemetrische Einrichtung zumindest bereichsweise im ersten Bereich angeordnet ist, und wobei an einem oberen bzw. freien Ende des ersten Bereichs der Drucksensor angeordnet ist, wobei insbesondere der Hohlkörper an dem oberen bzw. freien Ende des ersten Bereichs anschließt und den gasdichten Innenraum mit dem Drucksensor ausbildet oder zumindest teilweise begrenzt, oder wobei insbesondere das obere bzw,. freie Ende des ersten Bereichs durch eine gasdichte Membran abgeschlossen ist, die den gasdichten Innenraum zumindest bereichsweise begrenzt.

Diese Ausführungsform vereint in besonders vorteilhafter Weise die Funkwellenpermeabilität des ersten Bereichs mit einem ökonomischen und wirtschaftlichen Material, nämlich einem biokompatiblem Polymer. Durch die Anordnung der telemetrischen Einrichtung im ersten Bereich wird sichergestellt, dass die Übertragung der Messdaten gewährleistet ist. Dadurch dass das elastische Material, entweder in Form eines herausragenden Hohlkörpers oder aber in Form einer elastischen Membran am Ende des Körpers bzw. des ersten Bereichs angeordnet ist, ist eine vergleichsweise einfache Struktur des Körpers gewährleistet, was zur wirtschaftlichen Herstellung beiträgt.

Biokompatibel bedeutet in diesem Zusammenhang, dass das Polymer im Magen des Nutztieres keine Gift- oder Schadstoffe frei setzt und keine kontraindizierenden Reaktionen bei dem Nutztier auslöst.

Der obere Bereich des ersten Bereichs ist vorzugsweise als Endstück des Körpers geformt.

Vorteilhafterweise weist der zweite Bereich einen insbesondere zylinderförmigen Mantel auf, der auf einer Unterseite des ersten Bereichs einen Boden aufweist, wobei der Mantel insbesondere Edelstahl aufweist, und wobei der Mantel eine Aufnahme für eine Energiequelle für die telemetrische Einrichtung umfasst, wobei die Energiequelle insbesondere eine Batterie oder ein wiederaufladbarer Akku sein kann.

Diese Ausführungsform kann besonders vorteilhaft in Kombination mit dem ersten Bereich, der ein biokompatibles Polymer aufweist, ausgeführt werden. Der Edelstahl trägt insbesondere zur Stabilität, zur Verträglichkeit (weil inert) und zum Erreichen der hinreichend großen Dichte des Körpers bei. Weiterhin ist so eine sichere Aufnahme für die Energiequelle gewährleistet.

Das erfindungsgemäße Problem wird ausgehend vom gleichen Erfindungsgedanken, auch durch eine zweite Vorrichtung mit den folgenden Merkmalen realisiert.

Eine solche zweite Vorrichtung zur Erfassung der Magenmotilität eines Nutztieres, insbesondere eines Wiederkäuers, weist einen ersten Teilkörper und einen zweiten Teilkörper auf, wobei der erste Teilkörper mit dem zweiten Teilkörper über ein Gelenk verbunden ist, wobei die Vorrichtung einen Positionssensor aufweist, der dazu eingerichtet ist, die relative Lage des ersten Teilkörpers zum zweiten Teilkörper zu bestimmen, wobei der erste Teilkörper eine Vorrichtung gemäß den obigen Ausführungen umfasst.

Ein solches Gelenk kann beispielsweise ein Kugel oder aber auch ein scharnierartiges Gelenk sein. Im weitesten Sinne ist insbesondere jede flexible Verbindung, wie beispielsweise ein kurzes Band, des ersten Teilkörpers mit dem zweiten Teilkörper als Gelenk geeignet.

Diese zweite Vorrichtung bestimmt die Magenmotilität anhand der Bewegungen der zwei Teilkörper, und verwirklicht ebenso wie die oben offenbarte Vorrichtung ein Mittel zum Erfassen der Magenmotilität.

Insbesondere die Kombination der oben offenbarten Vorrichtung mit der zweiten Vorrichtung löst das erfindungsgemäße Problem mit verschiedenen und teilweise redundanten Mitteln, was zu einer besonders zuverlässigen Erfassung der Magenmotilität beiträgt.

Des Weiteren wird ein Verfahren zum Messen des Magendrucks mit der erfindungsgemäßen Vorrichtung offenbart, wobei das Verfahren die folgenden Schritte aufweist:
- Vorzugsweise orales Einführen der Vorrichtung in den Magen des Nutztieres, derart dass die Vorrichtung dauerhaft, insbesondere bis zum Tod oder operativen Entfernung, im Magen des Nutztieres verbleibt,
- Messen einer Zeitserie des Drucks,
- Übertragen der Messdaten aus dem Magen des Nutztieres heraus.

Das (insbesondere orale) Einführen der Vorrichtung in den Magen des Nutztieres muss nicht notwendiger Weise einen Schritt des Verfahrens darstellen. Das Verfahren kann auch zu einem Zeitpunkt einsetzen, zu dem die besagte Vorrichtung bereits eingeführt ist bzw. im Magen des Nutztieres vorhanden ist.

Weitere Merkmale und Vorteile der Erfindung werden nachfolgend anhand der Figurenbeschreibung von Ausführungsbeispielen erläutert. Es zeigen:
- Fig. 1: zwei Schnittansichten durch eine erfindungsgemäße Vorrichtung;
- Fig. 2: eine Schnittansicht durch eine erfindungsgemäße Vorrichtung mit einem Hohlkörper, und
- Fig. 3: eine Schnittansicht einer Modifikation der Ausführungsform gemäß Fig. 1.

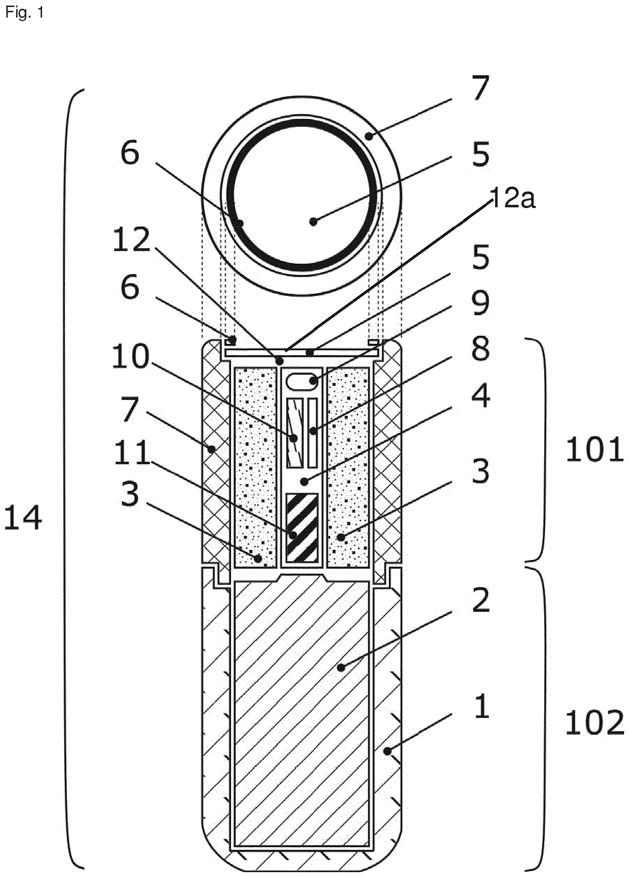

In Figur 1 ist eine erfindungsgemäße Vorrichtung in zwei Schnittansichten dargestellt. Die Vorrichtung weist einen insbesondere bolusförmigen Körper 14 - auch ggf. kurz Bolus genannt - auf. Der Körper 14 ist in einen ersten Bereich oder Abschnitt 101 und zweiten Bereich oder Abschnitt 102 unterteilt. Der erste Bereich 101 weist einen zylinderförmigen Mantel 7 aus POM auf. Der Mantel 7 umfasst dabei einen Füllkörper 3, in dem die Elektronik 4 des Körpers 14 angeordnet ist. Durch die Wahl des Polymers wird zum einen die Permeabilität für Funk- und Radiowellen erreicht und zum anderen ist dieses Material gleichzeitig biokompatibel.

Die Elektronik 4 umfasst dabei unter anderem eine telemetrische Einrichtung 8, einen Drucksensor 9 sowie einen Mikrocontroller 10 und ein Speichermedium 11. Der druckempfindliche Teil des Drucksensors liegt in einem gasdichten Innenraum 12 (oder kann mit diesem kommunizieren), dessen Wandung 12a zumindest teilweise ebenfalls aus dem Mantel 7 des ersten Bereichs 101 ausgebildet ist und dessen Decke (auch Teil der Wandung) von einer elastischen Membran 5 aus Silikonkautschuk gebildet wird. Der gasdichte Innenraum 12 liegt dabei an einem Ende des ersten Bereichs 101 des Körpers 14 und ist vorzugsweise stirnseitig am längserstreckten Körper 14 angeordnet.

Wenn sich die Membran 5, aufgrund einer Krafteinwirkung verformt, dann ändert sich der Druck in dem gasdichten Innenraum 12, was der Drucksensor 9 erfassen kann. Der Innenraum 12 wird mit einem Verschlussmittel in Form eines Rings 6, der die Membran 5 fixiert, abgeschlossen. Der Innenraum 12 ist somit gasdicht verschlossen.

Die Elektronik 4 ist intern so verschaltet, dass die Messdaten des Drucksensors 9 durch den Mikrocontroller 10 verarbeitet werden können, auf dem Speichermedium 11 abgespeichert werden können und mit der telemetrischen Einrichtung 8 an eine externe Basisstation gesendet werden können. Weiterhin kann eine Verarbeitung der Messdaten auch durch die telemetrische Einrichtung 8 selbst geschehen.

Der zweite Bereich 102 des Körpers14 schließt sich unterhalb des ersten Bereichs 101 an. Der zweite Bereich 102 weist einen zylinderförmigen Mantel 1 auf (z.B. aus einem Edelstahl), der eine Energiequelle zur Versorgung der Elektronik 4 in Form einer Batterie 2 umfasst.

Das Aktivieren der Elektronik 4 erfolgt mittels einer Selbsthalteschaltung, die mit einem Reed-Kontakt ausgelöst wird. Dazu schließt ein Magnet von außen den Reed-Kontakt und aktiviert so die Elektronik 4.

Die Elektronik 4 kann sich durch einen entsprechenden Pegel am Reset der Selbsthalteschaltung wieder ausschalten, was einen Testdurchlauf des gesamten Körpers 14 bzw. der Vorrichtung ermöglicht.

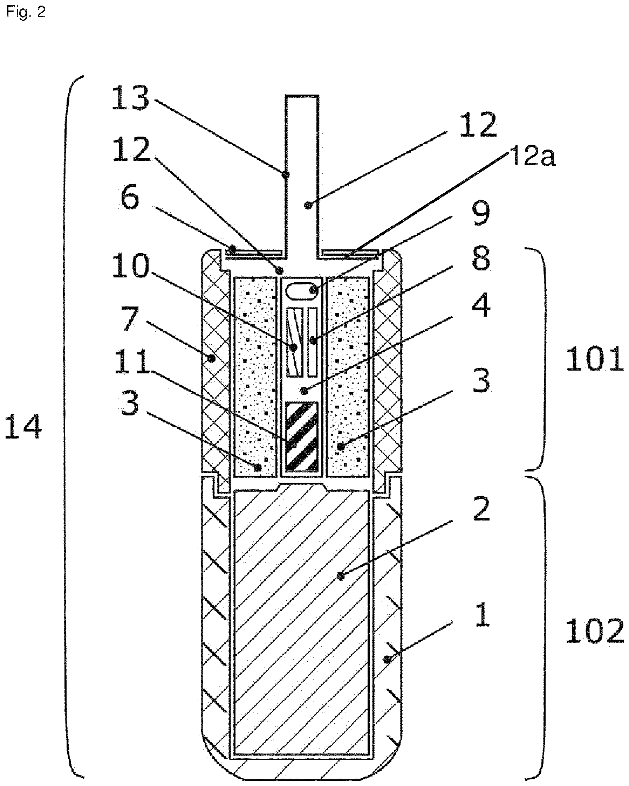

Im Unterschied zu Fig. 1 zeigt Fig. 2 eine Variante der Erfindung, bei der ein aus dem Körper 14 herausragender, elastischer Hohlkörper 13 aus z.B. Silikonkautschuk den gasdichten Innenraum 12 oder einen Teil dieses Innenraumes 12 ausbildet bzw. begrenzt und nicht die elastische Membran 5. Der Hohlkörper 13 ist hier als Sonde konfiguriert, die besonders vorteilhaft die Magenmotilität eines Nutztieres erfassen kann, da sie bevorzugt knickbar ausgeführt ist. Alle anderen Komponenten sind analog zu den Merkmalen, die bereits in Fig. 1 gezeigt sind, angeordnet bzw. ausgebildet.

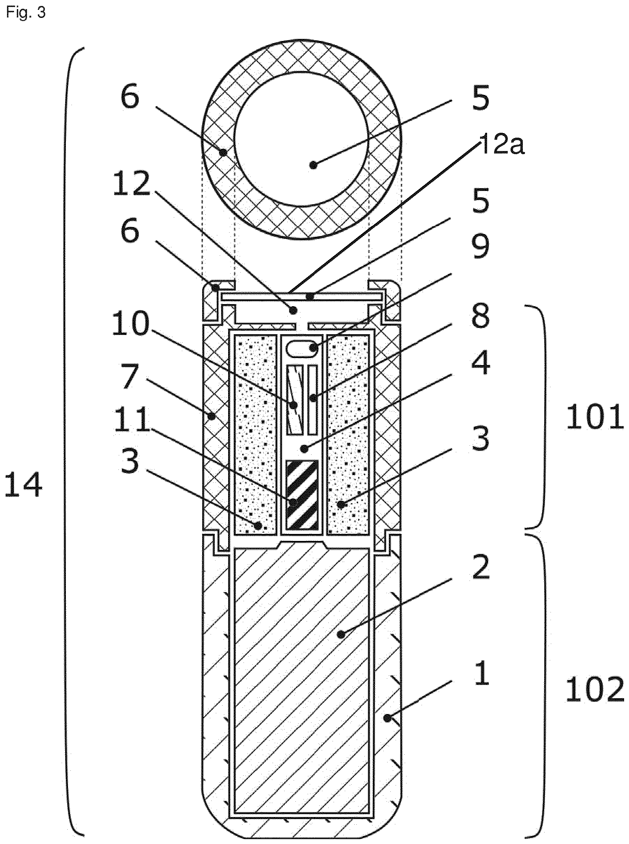

Eine weitere Ausgestaltung des Sensorsystems zeigt Figur 3. Dargestellt ist der Mantel 1, welcher die Energiequelle 2 schützend umschließt, der Füll- oder alternativ Massekörper 3, die Elektronik 4, die flexible Membran 5, sowie die Dichtungskappe 6, der Mantel 7, die telemetrische Einrichtung 8, der Drucksensor 9, wie auch der Mikrocontroller 10 und das Speichermedium 11, sowie der gasdichte Innenraum 12.

Im Unterschied zur Figur 1 ist hier das Verschlussmittel 6 als eine ringförmige Dichtungskappe 6 ausgebildet, die dazu konfiguriert ist, mit dem Körper 14, insbesondere dem Mantel 7 verschraubt zu werden, wobei die Membran 5 über dem Innenraum 12 fixiert wird. Hierzu drückt das Verschlussmittel 6, wenn es bestimmungsgemäß mit dem Körper 14 bzw. Mantel 7 verschraubt ist, die Membran 5 gegen eine umlaufende Anlagefläche des Mantels 7, wobei die Membran 5 am Körper 14 bzw. am Mantel 7 festgelegt wird und der Innenraum 12 gasdicht verschlossen wird.

Insbesondere drückt jene ringförmige Dichtungskappe 6 in einer Ausführungsform mit einem umlaufenden Bereich gegen einen umlaufenden Randbereich der insbesondere kreisförmigen Membran 5. Die Membran 5 steht hierbei nicht über die Dichtungskappe 6 in axialer Richtung des Körpers 14 hinaus, was der Membran 5 einen zusätzlichen Schutz verleiht.

### Weiterbildung der Erfindung

Eine Weiterbildung der Erfindung wird im Folgenden geschildert.

Der Betrieb von Vorrichtungen die für ihre Funktion elektrischen Strom benötigen, ist technisch bedingt, in seiner Laufzeit begrenzt (z.B. wenn die Energieversorgung über einen mobilen Energiespeicher, z.B. Batterie oder Akku, erfolgt). Langzeitmessungen bei denen die Vorrichtung, welche ein Sensor oder ein Sensorsystem sein kann, in einen lebenden Organismus, wie beispielsweise ein Rind, verbracht wird, bieten naturgemäß keinen physikalischen Zugriff mehr auf die Vorrichtung.

Ein Austausch der Energieversorgung zum Aufrechterhalten der Messaufgabe ist dann ausgeschlossen. Insbesondere bei sogenannten Pansen- Boli [OE 600 17 916 T2] [WO 2011/079338 A2] [GB 2 455 700 A], bei denen der Bolus aktiviert und sodann *per* os in den Pansen von vorzugsweise Kühen eingeführt wird, besteht nach dem Einsetzen des Bolus keine Möglichkeit mehr, diesen zwecks Batterie- oder Sensorwechsel auszutauschen.

Eine unzureichende Lösung des Problems ist, das mehrfache Nachlegen eines frischen Bolus mit dem Nachteil, dass dies nur begrenzt oft je Lebewesen bzw. Einzeltier möglich ist. Auch ein Ersetzen eines erschöpften Wirkstoffvorrates oder ein Austausch bei einer Fehlfunktion ist bislang nicht möglich. Dieses Problem wird von der Weiterbildung der Erfindung adressiert.

Zusätzlich ergäbe sich durch eine Wiederverwendung der Vorrichtung durch Etablierung eines Kreislaufsystems, bei dem die Vorrichtung wieder aufbereitet wird, eine nachhaltige und wirtschaftliche Möglichkeit, die Kosten gering zu halten.

Auch der immer mehr in den Fokus rückende Umweltschutz wäre durch einen planvollen Ressourcenumgang berücksichtigt.

### Aufgabe der Weiterbildung der Erfindung

Das Einsatzgebiet der Vorrichtung ist im Bereich des Gesundheitsmonitorings oder auch von Wirkstoff- Verabreichungen bei Lebewesen, vorzugsweise Nutztieren, angesiedelt. Da die Vorrichtung austauschbar ist, sind die Laufzeiten nur von der Möglichkeit die Vorrichtung auszutauschen, limitiert.

Pansenboli, wie sie beispielsweise für pH- Messungen im Pansen bei Kühen im Einsatz sind DE 60017916 T2, WO 2011/079338 A2, und GB 2455700 A müssen eine minimale Dichte von 2,3 g/cm³ aufweisen, damit sie dauerhaft im Netzmagen (Pansen) verbleiben. Bei geringerer Dichte wandern diese weiter im Verdauungstrakt und verlassen diesen schließlich auf natürlichem Wege und werden ausgeschieden.

Wenn demnach ein solcher Sensor beim Einsetzen bzw. während des Betriebs die geforderte Mindestdichte aufweist und auf ein bestimmtes Signal hin, sich seine Dichte oder Masse verringert, erhält man eine Vorrichtung, welche sich auf Wunsch aus dem Organismus auf natürlichem Wege selbst entfernt. Diese kann dann durch eine neue ersetzt werden, was idealerweise nahtlos erfolgen kann.

Die Veränderung an der Vorrichtung kann in einer bevorzugten Ausführung durch die Bewegung eines Kolbens, welcher eine im Bolus befindliche Lösung herausbewegt, erfolgen. Die Folge dieses Herausbewegen einer Lösung durch die Kolbenbewegung wäre eine Verringerung des Gesamtgewichts des Bolus. Zusätzlichen Auftrieb erführe die Vorrichtung durch die Raum- bzw. Druckveränderung des vom Kolben freigegebenen Kolbenraumes.

Die Veränderung an der Vorrichtung kann in einer weiteren Variante der Ausführung durch das Abwerfen eines Ballastkörpers erfolgen.

Auch das Ablassen einer Schwerflüssigkeit wie z. B. das untoxische Natriumpolywolframat würde zu einer Masseverringerung führen. Die Vorrichtung wird durch jede der aufgeführten Maßnahmen für sich, entsprechend leichter und kann den natürlichen Weg der Ausscheidung nehmen, da die geforderte Dichte zum Verbleib nicht mehr vorliegt.

## Patentansprüche

1. Vorrichtung zur Erfassung des Magendrucks eines Nutztieres, insbesondere eines Wiederkäuers, aufweisend einen Körper (14), wobei der Körper (14) die folgenden Komponenten aufweist:
- einen Drucksensor (9),
- eine telemetrische Einrichtung (8) zum Übertragen von Messwerten des Drucksensors (9),
- einen gasdichten Innenraum (12), wobei eine Wandung (12a) des gasdichten Innenraums (12) einen elastischen Bereich (5, 13) aufweist, der durch den Magendruck eines Nutztiers verformbar ist, und wobei der Drucksensor (9) dazu ausgebildet ist, den Druck im gasdichten Innenraum (12) zu erfassen,
**dadurch gekennzeichnet,**
**dass** der Körper (14) eine mittlere Dichte von mehr als 2,3 g/cm³ aufweist, so dass gewährleistet ist, dass die Vorrichtung dauerhaft im Magen des Nutztieres verbleibt, wenn sie dem Nutztier appliziert wurde und dass die Vorrichtung einen Bewegungssensor umfasst, der dazu eingerichtet ist, die Bewegungsaktivität des Nutztiers zu erfassen.

2. Vorrichtung nach Anspruch 1, **dadurch gekennzeichnet, dass** der Körper (14) eine mittlere Dichte von weniger als 3,5 g/cm³ aufweist, wobei die Dichte besonders bevorzugt bei 2,8 g/ cm³ liegt.

3. Vorrichtung nach Anspruch 1 oder 2, **dadurch gekennzeichnet, dass** der Körper (14) ein Volumen zwischen 50 cm³ und 250 cm³, bevorzugt zwischen 70 cm³ und 160 cm³, aufweist, wobei das Volumen besonders bevorzugt 150 cm³ beträgt.

4. Vorrichtung nach einem der vorhergehenden Ansprüche, **dadurch gekennzeichnet, dass** der Bereich (5) als eine elastische Membran (5) ausgebildet ist.

5. Vorrichtung nach einem der vorhergehenden Ansprüche, wobei jener Bereich der Wandung (12a) des gasdichten Innenraums (12) durch einen aus dem Körper (14) herausragenden, elastischen Hohlkörper (13) gebildet ist, wobei der Hohlkörper (13) durch auf ihn einwirkende Kräfte im Magen des Nutztieres verformbar ist.

6. Vorrichtung nach Anspruch 4 oder 5, **dadurch gekennzeichnet, dass** die Membran (5) oder der Hohlkörper (13) mittels eines ringförmigen Verschlussmittels (6) am Körper (14) fixierbar sind.

7. Vorrichtung nach einem der Ansprüche 5 bis 6, **dadurch gekennzeichnet, dass** der Hohlkörper (13) schlauchförmig, insbesondere zylindrisch, ausgebildet ist.

8. Vorrichtung nach einem der vorhergehenden Ansprüche, **dadurch gekennzeichnet, dass** die Vorrichtung einen Temperatursensor aufweist, der dazu eingerichtet ist, eine Magentemperatur im Nutztier zu erfassen, wenn die Vorrichtung in dem Magen des Nutztiers angeordnet ist, und/oder wobei der Bewegungssensor einen Beschleunigungssensor aufweist.

9. Vorrichtung nach einem der vorhergehenden Ansprüche, **dadurch gekennzeichnet, dass** die telemetrische Einrichtung (8) dazu eingerichtet ist, die Messdaten des Drucksensors (9) und insbesondere die Messdaten des Temperatursensors und/oder des Bewegungssensors zu verarbeiten und die verarbeiteten Daten an einen Empfänger zu senden.

10. Vorrichtung nach einem der vorhergehenden Ansprüche, **dadurch gekennzeichnet, dass** der Körper (14) einen Mikrocontroller (10) und einen Datenspeicher (11) aufweist, auf dem die Messdaten oder die verarbeiteten Messdaten abspeicherbar sind.

11. Vorrichtung nach einem der vorhergehenden Ansprüche, **dadurch gekennzeichnet, dass** der Körper (14) einen ersten Bereich (101) und einen zweiten Bereich (102) aufweist, wobei der erste Bereich (101) des Körpers (14) radio- und funkwellenpermeabel ist, und wobei der zweite Bereich (102) zusätzlich so beschaffen ist, dass der Körper (14) zumindest die mittlere Dichte aufweist.

12. Vorrichtung nach Anspruch 11, **dadurch gekennzeichnet, dass** der erste Bereich (101) einen insbesondere zylinderförmigen Mantel (7) aus einem biokompatiblen Polymer aufweist, wobei die telemetrische Einrichtung (8) im ersten Bereich (101) angeordnet ist, und wobei an einem oberen Ende des ersten Bereichs (101) der Drucksensor (9) angeordnet ist, wobei insbesondere der Hohlkörper (13) an dem oberen Ende des ersten Bereichs (101) anschließt und den gasdichten Innenraum (12) zumindest teilweise begrenzt, oder wobei insbesondere der erste Bereich (101) an dem oberen Ende durch die Membran (5) abgeschlossen ist, die den gasdichten Innenraum (12) zumindest teilweise begrenzt.

13. Vorrichtung nach einem der Ansprüche 11 oder 12, **dadurch gekennzeichnet, dass** der zweite Bereich (102) einen insbesondere zylinderförmigen Mantel (1) aufweist, der auf einer Unterseite des ersten Bereichs (101) einen Boden aufweist, wobei der Mantel (1) insbesondere Edelstahl aufweist, und wobei der Mantel (1) eine Aufnahme für eine Energiequelle (2) für die telemetrische Einrichtung (8) umfasst, wobei die Energiequelle (2) insbesondere eine Batterie oder ein wiederaufladbarer Akku sein kann.

14. Vorrichtung zur Erfassung der Magenmotilität eines Nutztieres, insbesondere eines Wiederkäuers, aufweisend einen ersten Teilkörper und einen zweiten Teilkörper, wobei der erste Teilkörper mit dem zweiten Teilkörper über ein Gelenk verbunden ist, wobei die Vorrichtung einen Positionssensor aufweist, der dazu eingerichtet ist, die relative Lage des ersten Teilkörpers zum zweiten Teilkörper zu bestimmen, wobei der erste Teilkörper eine Vorrichtung nach einem der vorhergehenden Ansprüche aufweist.

## Claims

1. A device for detecting the gastric pressure of a livestock animal, in particular of a ruminant, having a body (14), the body (14) having the following components:
- a pressure sensor (9),
- a telemetric device (8) for transmitting measured values from the pressure sensor (9),
- a gas-tight interior (12), one wall (12a) of the gas-tight interior (12) having a resilient region (5, 13) which is deformable by the gastric pressure of a livestock animal, and the pressure sensor (9) being formed to detect the pressure in the gas-tight interior (12),
**characterised in that**
the body (14) has an average density of greater than 2.3 g/cm³ so as to ensure that the device remains permanently in the stomach of the livestock animal when it has been administered to the livestock animal and **in that** the device comprises a motion sensor, wherein the motion sensor is configured to detect the movement activity of the livestock animal.

2. The device according to claim 1, **characterised in that** the body (14) has an average density of less than 3.5 g/cm³, wherein the density is particularly preferably 2.8 g/cm³.

3. The device according to claim 1 or 2, **characterised in that** the body (14) has a volume of between 50 cm³ and 250 cm³, preferably between 70 cm³ and 160 cm³, wherein the volume particularly preferably amounts to 150 cm³.

4. The device according to any one of the preceding claims, **characterised in that** the region (5) is formed as a resilient membrane (5).

5. The device according to any one of the preceding claims, wherein said region of the wall (12a) of the gas-tight interior (12) is formed by a resilient hollow body (13) projecting out from the body (14), wherein the hollow body (13) is deformable by forces acting thereon in the stomach of the livestock animal.

6. The device according to claim 4 or 5, **characterised in that** the membrane (5) or the hollow body (13) are fixable to the body (14) by means of an annular closure means (6).

7. The device according to any one of claims 5 to 6, **characterised in that** the hollow body (13) is formed tubularly, particularly cylindrically.

8. The device according to any one of the preceding claims, **characterised in that** the device has a temperature sensor which is configured to detect a gastric temperature in the livestock animal when the device is arranged in the stomach of the livestock animal, and/or wherein the motion sensor comprises an acceleration sensor.

9. The device according to any one of the preceding claims, **characterised in that** the telemetric device (8) is configured to process the measurement data of the pressure sensor (9) and in particular the measurement data of the temperature sensor and/or the motion sensor and to send the processed data to a receiver.

10. The device according to any one of the preceding claims, **characterized in that** the body (14) has a microcontroller (10) and a data memory (11) in which the measurement data or the processed measurement data are storable.

11. The device according to any one of the preceding claims, **characterised in that** the body (14) has a first region (101) and a second region (102), wherein the first region (101) of the body (14) is radio wave transmissive and wherein the second region (102) is additionally constructed such that the body (14) has at least the average density.

12. The device according to claim 11, **characterised in that** the first region (101) has an in particular cylindrical casing (7) of a biocompatible polymer, wherein the telemetric device (8) is arranged in the first region (101) and wherein the pressure sensor (9) is arranged at an upper end of the first region (101), wherein in particular the hollow body (13) adjoins the upper end of the first region (101) and at least in part bounds the gas-tight interior (12), or wherein in particular the first region (101) is closed at the upper end by the membrane (5) which at least in part bounds the gas-tight interior (12).

13. The device according to either one of claims 11 or 12, **characterised in that** the second region (102) has an in particular cylindrical casing (1) which has a bottom on an underside of the first region (101), wherein the casing (1) in particular comprises stainless steel and wherein the casing (1) comprises a receptacle for an energy source (2) for the telemetric device (8), wherein the energy source (2) may in particular be a primary or a rechargeable battery.

14. A device for detecting the gastric motility of a livestock animal, in particular of a ruminant, having a first subbody and a second subbody, wherein the first subbody is connected to the second subbody via a joint, wherein the device has a position sensor which is configured to determine the relative position of the first subbody to the second subbody,wherein the first subbody in particular comprises a device according to any one of the preceding claims.

## Revendications

1. Dispositif de détection de la pression gastrique d'un animal de rente, notamment d'un ruminant, présentant un corps (14), dans lequel le corps (14) présente les composants suivants :
- un capteur de pression (9),
- une installation télémétrique (8) pour la transmission de valeurs de mesure du capteur de pression (9),
- un espace interne étanche au gaz (12), dans lequel une paroi (12a) de l'espace interne étanche au gaz (12) présente une région élastique (5, 13) qui peut être déformée par la pression gastrique d'un animal de rente, et dans lequel le capteur de pression (9) est réalisé pour détecter la pression dans l'espace interne étanche au gaz (12),
**caractérisé en ce**
**que** le corps (14) présente une densité moyenne de plus de 2,3 g/cm³ de sorte qu'il est garanti que le dispositif reste durablement dans l'estomac de l'animal de rente lorsqu'il a été appliqué à l'animal de rente et que le dispositif comprend un capteur de mouvement qui est configuré pour détecter l'activité de mouvement de l'animal de rente.

2. Dispositif selon la revendication 1, **caractérisé en ce que** le corps (14) présente une densité moyenne de moins de 3,5 g/cm³, dans lequel la densité se situe de manière particulièrement préférée à 2,8 g/cm³.

3. Dispositif selon la revendication 1 ou 2, **caractérisé en ce que** le corps (14) présente un volume compris entre 50 cm³ et 250 cm³, de préférence entre 70 cm³ et 160 cm³, dans lequel le volume se monte de manière particulièrement préférée à 150 cm³.

4. Dispositif selon une des revendications précédentes, **caractérisé en ce que** la région (5) est réalisée en tant que membrane élastique (5).

5. Dispositif selon une des revendications précédentes, **caractérisé en ce que** cette région-là de la paroi (12a) de l'espace interne étanche au gaz (12) est formée par un corps creux élastique (13) dépassant du corps (14), dans lequel le corps creux (13) peut être déformé par des forces agissant sur lui dans l'estomac de l'animal de rente.

6. Dispositif selon la revendication 4 ou 5, **caractérisé en ce que** la membrane (5) ou le corps creux (13) peut être fixé(e) au corps (14) au moyen d'un moyen de fermeture annulaire (6).

7. Dispositif selon une des revendications 5 à 6, **caractérisé en ce que** le corps creux (13) est réalisé en forme de tuyau, notamment de manière cylindrique.

8. Dispositif selon une des revendications précédentes, **caractérisé en ce que** le dispositif présente un capteur de température qui est configuré pour détecter une température gastrique dans l'animal de rente lorsque le dispositif est disposé dans l'estomac de l'animal de rente, et/ou dans lequel le capteur de mouvement présente un capteur d'accélération.

9. Dispositif selon une des revendications précédentes, **caractérisé en ce que** l'installation télémétrique (8) est configurée pour traiter les données de mesure du capteur de pression (9) et notamment les données de mesure du capteur de température et/ou du capteur de mouvement, et envoyer les données traitées à un récepteur.

10. Dispositif selon une des revendications précédentes, **caractérisé en ce que** le corps (14) présente un microcontrôleur (10) et une mémoire de données (11) sur laquelle les données de mesure ou les données de mesure traitées peuvent être mémorisées.

11. Dispositif selon une des revendications précédentes, **caractérisé en ce que** le corps (14) présente une première région (101) et une seconde région (102), dans lequel la première région (101) du corps (14) est perméable aux ondes radioélectriques, et dans lequel la seconde région (102) est en outre pourvue de telle sorte que le corps (14) présente au moins la densité moyenne.

12. Dispositif selon la revendication 11, **caractérisé en ce que** la première région (101) présente une enveloppe notamment cylindrique (7) en un polymère biocompatible, dans lequel l'installation télémétrique (8) est disposée dans la première région (101), et dans lequel le capteur de pression (9) est disposé à une extrémité supérieure de la première région (101), dans lequel le corps creux (13) est notamment relié à l'extrémité supérieure de la première région (101) et limite au moins partiellement l'espace interne étanche au gaz (12), ou dans lequel la première région (101) est notamment terminée par la membrane (5) à l'extrémité supérieure, laquelle limite au moins partiellement l'espace interne étanche au gaz (12).

13. Dispositif selon une des revendications 11 ou 12, **caractérisé en ce que** la seconde région (102) présente une enveloppe notamment cylindrique (1) qui présente un fond sur un côté inférieur de la première région (101), dans lequel l'enveloppe (1) présente notamment de l'acier inoxydable, et dans lequel l'enveloppe (1) comprend un accueil pour une source d'énergie (2) pour l'installation télémétrique (8), dans lequel la source d'énergie (2) peut notamment être une batterie ou une batterie rechargeable.

14. Dispositif de détection de la motilité gastrique d'un animal de rente, notamment d'un ruminant, présentant un premier corps partiel et un second corps partiel, dans lequel le premier corps partiel est connecté au second corps partiel par le biais d'une articulation, dans lequel le dispositif présente un capteur de position qui est configuré pour déterminer la position relative du premier corps partiel par rapport au second corps partiel, dans lequel le premier corps partiel présente un dispositif selon une des revendications précédentes.
